(19) **Europäisches Patentamt / European Patent Office / Office européen des brevets**

(11) **EP 4 487 697 A1**

(12) # EUROPEAN PATENT APPLICATION
published in accordance with Art. 153(4) EPC

(43) Date of publication:
**08.01.2025 Bulletin 2025/02**

(21) Application number: **23763570.1**

(22) Date of filing: **03.03.2023**

(51) International Patent Classification (IPC):
*A23L 29/281* (2016.01)    *A23C 9/137* (2006.01)
*A23C 13/16* (2006.01)    *A23J 3/00* (2006.01)
*A23J 3/16* (2006.01)    *A23L 5/00* (2016.01)
*A23L 9/20* (2016.01)    *A23L 27/60* (2016.01)
*C12N 9/10* (2006.01)    *C12P 21/04* (2006.01)

(52) Cooperative Patent Classification (CPC):
**A23C 9/137; A23C 13/16; A23J 3/00; A23J 3/16;
A23L 5/00; A23L 9/20; A23L 27/60; A23L 29/281;
C07K 7/50; C12N 9/10**

(86) International application number:
**PCT/JP2023/008032**

(87) International publication number:
**WO 2023/167313 (07.09.2023 Gazette 2023/36)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **03.03.2022 JP 2022032720**

(71) Applicant: AJINOMOTO CO., INC.
**Chuo-ku
Tokyo 104-8315 (JP)**

(72) Inventors:
• **AOYAMA, Hiroaki**
  **Kawasaki-shi, Kanagawa 210-8681 (JP)**
• **KANEDA, Isamu**
  **Ebetsu-shi, Hokkaido 069-8501 (JP)**
• **SOGA, Takanori**
  **Kawasaki-shi, Kanagawa 210-8681 (JP)**
• **KAMIMURA, Yuya**
  **Kawasaki-shi, Kanagawa 210-8681 (JP)**

(74) Representative: **Strehl Schübel-Hopf & Partner
Maximilianstrasse 54
80538 München (DE)**

(54) **METHOD FOR MANUFACTURING FLUID GEL**

(57) The present invention provides a method for producing a fluid gel, including stirring a solution containing a protein and transglutaminase and the like.

[Fig. 1]

EP 4 487 697 A1

## Description

[Technical Field]

The present invention relates to a fluid gel which can impart stickiness-free viscosity and a rich taste, good melt in mouth, foaming property, and the like to semi-solid foods, and which itself is superior in emulsifying property, and a production method thereof, emulsifiers and semi-solid foods containing the fluid gel, and the like.

[Background Art]

Generally, in semi-solid foods such as yoghurt, mayonnaise, whipped cream, and the like, thickeners such as saccharides, starch, and the like are added to increase the viscosity. When, for example, a thickener is added to low-fat mayonnaise, the melt in mouth becomes poor, giving rise to a sticky texture. In recent years, on the other hand, due to changes in consumer needs, there has been an increasing demand for alternative foods such as low-fat and low-calorie foods and foods that use plant proteins instead of animal proteins. In those alternative foods, one of the goals is provision of properties similar to those of the original foods. Also in semi-solid foods, for example, since low-fat mayonnaise and mayonnaise made with plant proteins instead of animal proteins are too thin, thickeners, emulsifiers, and the like are added, but the addition of these causes a sticky texture. In addition, whipped cream made with vegetable fat does not foam well as compared with whipped cream made with fresh cream. Therefore, there is a demand for components, compositions, and the like that can impart semi-solid foods with viscosity free of stickiness, foaming property, and the like.

For property improvement in semi-solid foods, a creamer containing sugar, fat, and a protein having a spherical protein denaturation degree within a specific range, and having a viscosity within a specific range is known as a creamer that can achieve long-term stability, high whitening ability, and a pleasant mouthfeel (Patent Literature 1). In addition, an edible composition containing water, a fat phase, microgel particles, a stabilizing emulsifier, and an unstabilizing emulsifier, which has an elastic modulus within a specific range, has properties similar to those of mayonnaise or a spread, and can be spooned or spread is known (Patent Literature 2). Also, a method for producing ice cream and the like having a rich and smooth texture is known, in which a frozen gel obtained by heating and then freezing a whey protein concentrate, which is a protein gel, is used as a fat substitute (Patent Literature 3). However, none of these provide a satisfactory balance of stickiness, viscosity, rich taste, melt in mouth, and the like, or improve the balance of these.

On the other hand, methods using enzymes have been developed as a technique for modifying foods. For example, transglutaminase is an enzyme that catalyzes the cross-linking of proteins and is widely used as an adhesive enzyme for food materials. In addition, protein gelation by transglutaminase is known (Patent Literature 4, Patent Literature 5).

As methods for producing food gels, a method including dissolving agar or the like in hot water and cooling the solution while stirring, a method including heating protein such as whey while stirring, and the like are known.

[Citation List]

[Patent Literature]

[Patent Literature 1]
JP 2014-512193 A
[Patent Literature 2]
JP 2003-506061 A
[Patent Literature 3]
JP H03-280834 A
[Patent Literature 4]
JP S64-27471 A
[Patent Literature 5]
JP H06-113844 A

[Summary of Invention]

[Technical Problem]

The present invention aims to provide a fluid gel which can impart stickiness-free viscosity and a rich taste, good melt in mouth, foaming property, and the like to semi-solid foods, and which itself is superior in emulsifying property, and a production method thereof, emulsifiers and semi-solid foods containing the fluid gel, and the like.

[Solution to Problem]

The present inventors have conducted intensive studies of the above-mentioned problem and found that a fluid gel can be obtained by stirring a solution containing a protein and transglutaminase, and that addition of this fluid gel to low-fat mayonnaise, whipped cream using vegetable fat, and the like can provide properties closer to those of full-fat mayonnaise and whipped cream produced by stirring high-fat raw milk, respectively; specifically, viscosity and texture that are free of stickiness, good foaming property, and good melt in mouth, and have completed the present invention by conducting further studies based on such finding.

Accordingly, the present invention provides the following.

[1] A method for producing a fluid gel, comprising stirring a solution comprising a protein and transglutaminase.

[2] The method for producing a fluid gel of the above-mentioned [1], wherein a temperature during the stirring is a temperature at which the protein does not denature.

[3] The method for producing the fluid gel of the above-mentioned [1] or [2], wherein a stirring speed during the stirring is 100 rpm to 30,000 rpm.

[4] The method for producing a fluid gel of any of the above-mentioned [1] to [3], wherein the transglutaminase is used in an amount of 0.001 U to 1,000 U per 1 g of the protein.

[5] The method for producing a fluid gel of any of the above-mentioned [1] to [4], wherein the transglutaminase is added to the solution comprising the protein while stirring.

[6] The method for producing a fluid gel of any of the above-mentioned [1] to [5], wherein the protein is a plant protein.

[7] The method for producing a fluid gel of any of the above-mentioned [1] to [5], wherein the protein is an animal protein.

[8] A fluid gel comprising a protein treated with transglutaminase, the fluid gel having a median diameter of 0.1 $\mu$m to 1000 $\mu$m.

[9] A fluid gel comprising a protein treated with transglutaminase and satisfying one or more of the following properties at 25°C:

(1) coordination number in an angular frequency range of 0.1 to 100 rad/sec: 0.1 to 20
(2) gel strength in an angular frequency range of 0.1 to 100 rad/sec: 0.1 Pas$^{1/z}$ to 100 Pas$^{1/z}$
(3) viscosity coefficient in a shear rate range of 1 to 100/sec: 0.1 Pas$^n$ to 100 Pas$^n$.

[10] The fluid gel of the above-mentioned [9], having a median diameter of 0.1 $\mu$m to 1000 $\mu$m.

[11] An emulsifier comprising the fluid gel of any of the above-mentioned [8] to [10].

[12] A semi-solid food comprising the fluid gel of any of the above-mentioned [8] to [10].

[13] The semi-solid food of the above-mentioned [12], which is yoghurt, mayonnaise or whipped cream.

[14] A method for producing a semi-solid food, comprising mixing the fluid gel of any of [8] to [10] with a raw material for a semi-solid food.

[15] The production method of the above-mentioned [14], wherein the semi-solid food is yoghurt, mayonnaise or whipped cream.

[16] A method for improving the property of a semi-solid food, comprising mixing the fluid gel of any of the above-mentioned [8] to [10] with a raw material for a semi-solid food.

[Advantageous Effects of Invention]

The present invention can provide a fluid gel which can impart good properties such as stickiness-free viscosity and rich taste, good melt in mouth, foaming property, and the like to semi-solid foods, and which itself is superior in emulsifying property.

[Brief Description of Drawings]

[Fig. 1]

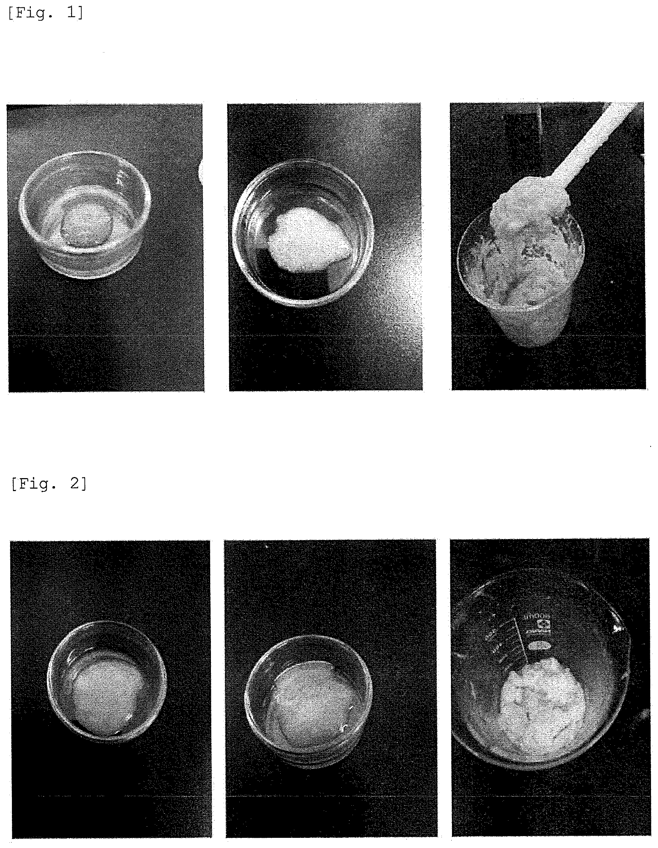

Fig. 1 is a photograph of each mixture obtained in Example 2. The photographs on the left, center, and right are photographs of the mixtures obtained when 1.22 U/g, 3.66 U/g, and 6.10 U/g of transglutaminase were added to the soy protein solution, respectively.

[Fig. 2]

Fig. 2 is a photograph of each mixture obtained in Example 3. The photographs on the left, center, and right are photographs of the mixtures obtained when 1.22 U/g, 3.66 U/g, and 6.10 U/g of transglutaminase were added to the soy protein solution, respectively.

[Fig. 3]

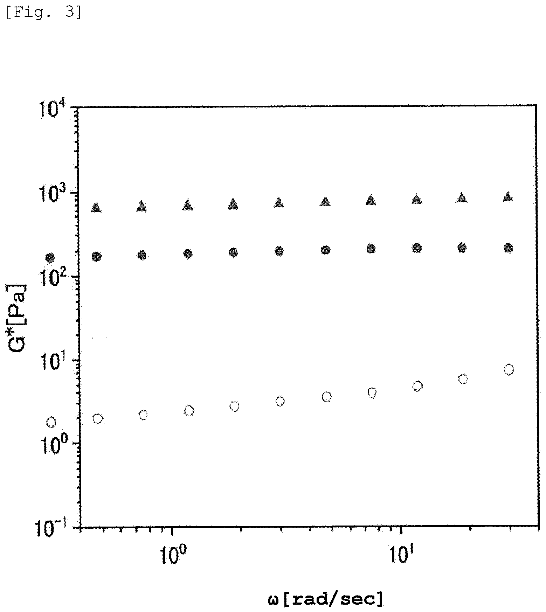

Fig. 3 is a graph showing the complex modulus of each fluid gel measured in Experimental Example 1. The vertical axis represents the complex modulus ($G^*$[Pa]), and the horizontal axis represents the angular frequency ($\omega$[rad/sec]). In addition, O, • and ▲ indicate data for fluid gels obtained using solutions containing transglutaminase and 5 wt%, 6 wt%, and 7 wt% soy protein, respectively.

[Fig. 4]

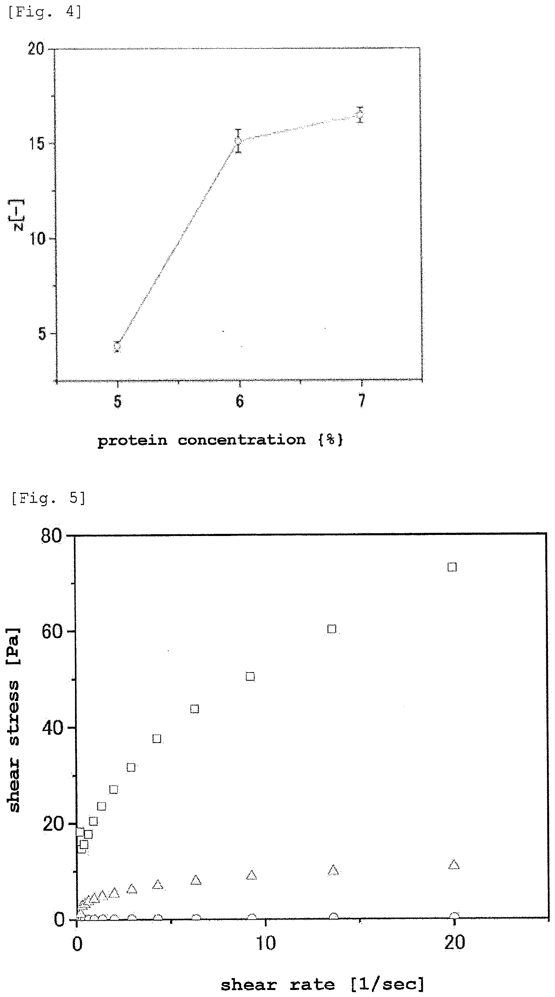

Fig. 4 is a graph showing the coordination number of each fluid gel measured in Experimental Example 1. The vertical axis represents the coordination number ($z$[-]), and the horizontal axis represents the protein concentration (wt%).

[Fig. 5]

Fig. 5 is a graph showing the shear stress, measured in Experimental Example 2, of each fluid gel obtained in Example 2. The vertical axis.represents the shear stress (shear stress [Pa]), and the horizontal axis represents the shear rate (1/sec) during steady-flow viscosity measurement. In addition, O, $\Delta$ and □ indicate data for fluid gels obtained by adding 0 U/g, 1.22 U/g, and 3.66 U/g of transglutaminase to soy protein solutions, respectively.

[Fig. 6]

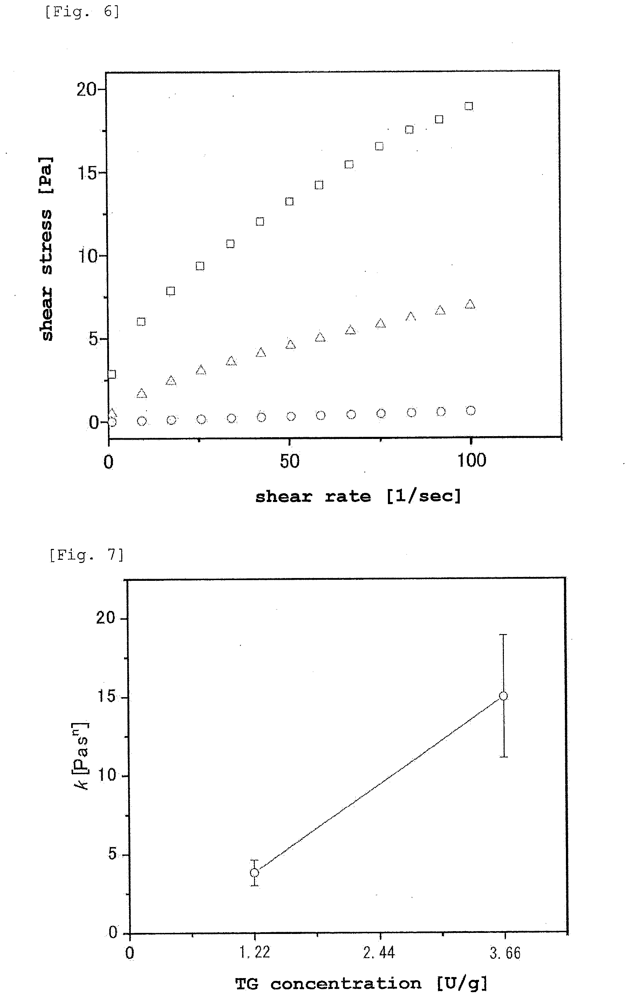

Fig. 6 is a graph showing the shear stress, measured in Experimental Example 2, of each fluid gel obtained in Example 3. The vertical axis represents the shear stress (shear stress [Pa]), and the horizontal axis represents the shear rate (1/sec) during steady-flow viscosity measurement. In addition, ○, $\Delta$ and □ indicate data for fluid gels obtained by adding 0 U/g, 1.22 U/g, and 3.66 U/g of transglutaminase to soy protein solutions, respectively.

[Fig. 7]

Fig. 7 is a graph showing the viscosity coefficient of each fluid gel obtained under alkaline conditions and measured in Experimental Example 3. The vertical axis represents the viscosity coefficient ($k$[Pas$^n$]) and the horizontal axis represents the transglutaminase concentration (U/g).

[Fig. 8]

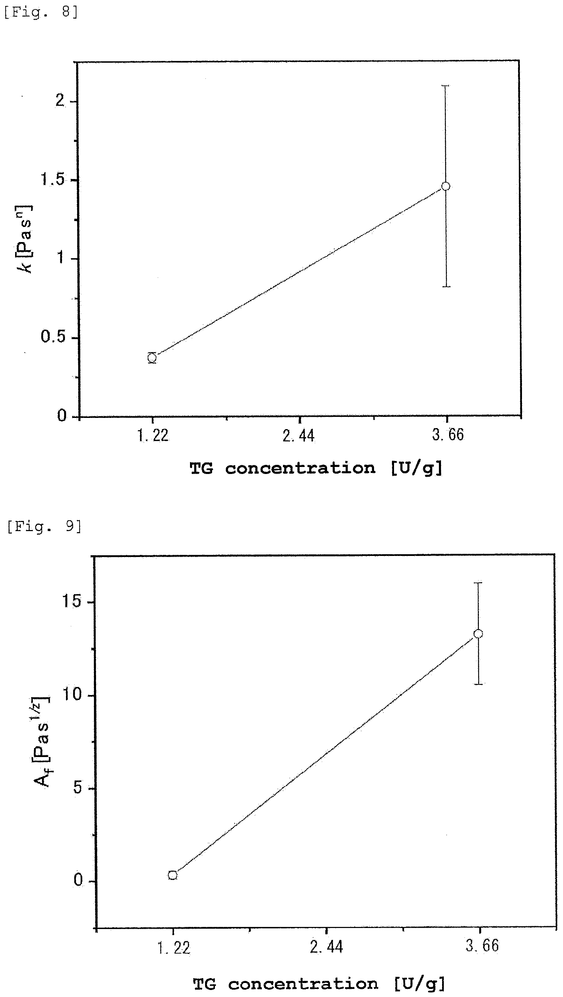

Fig. 8 is a graph showing the viscosity coefficient of each fluid gel obtained under neutral conditions and measured in Experimental Example 3. The vertical axis represents the viscosity coefficient ($k$[Pas$^n$]) and the horizontal axis represents the transglutaminase concentration (U/g).

[Fig. 9]

Fig. 9 is a graph showing the gel strength of each fluid gel produced under alkaline conditions and measured in Experimental Example 4. The vertical axis represents the gel strength ($A_f$[Pas$^{1/z}$]) and the horizontal axis represents the transglutaminase concentration (U/g).

[Fig. 10]

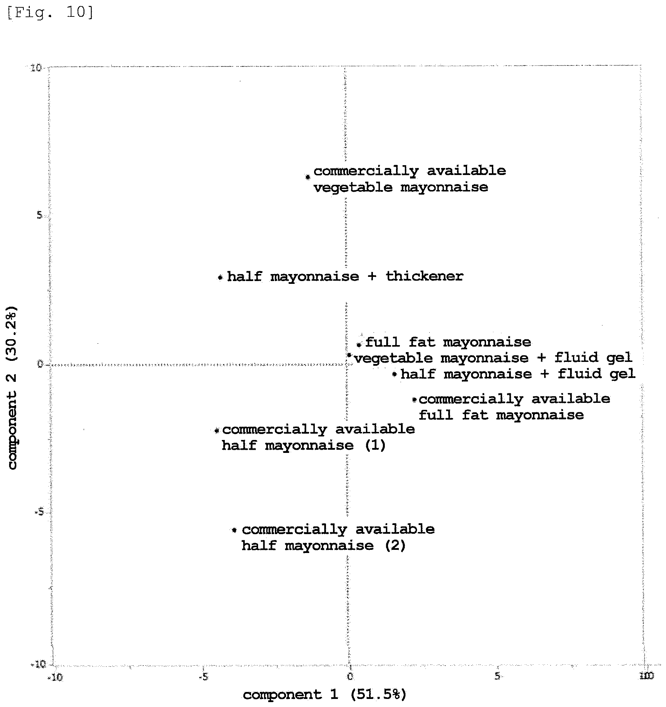

Fig. 10 is a score plot obtained by performing principal component analysis on the results of dynamic viscoelasticity, measured in Experimental Example 5, of each mayonnaise obtained in Example 4 and each commercially available mayonnaise. The horizontal axis represents the first principal component (component 1), the vertical axis represents the second principal component (component 2), and the figures in respective parentheses represent the contribution rate of each component.

[Fig. 11]

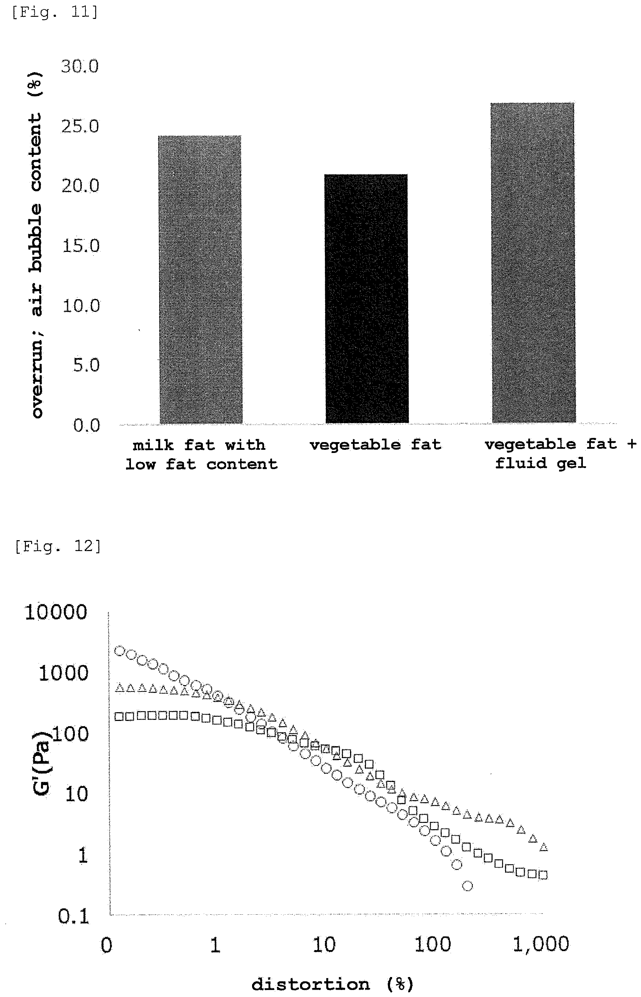

Fig. 11 is a graph showing the overrun (air bubble content) of each whipped cream obtained in Example 5. The vertical axis represents overrun (air bubble content) (%), and the horizontal axis represents the sample name.

[Fig. 12]

Fig. 12 is a graph showing the dynamic viscoelasticity, measured in Experimental Example 6, of each whipped cream obtained in Example 5. The vertical axis represents storage modulus ($G'$[Pa]), and the horizontal axis represents strain (%). In addition, O, $\Delta$ and □ respectively indicate data for whipped cream prepared from fresh cream (fresh cream), whipped cream prepared from vegetable fat (vegetable fat), and whipped cream prepared from a mixture in which fluid gel obtained from a solution containing 5 wt% soy protein and transglutaminase was added to vegetable fat (vegetable fat + fluid gel).

[Fig. 13]

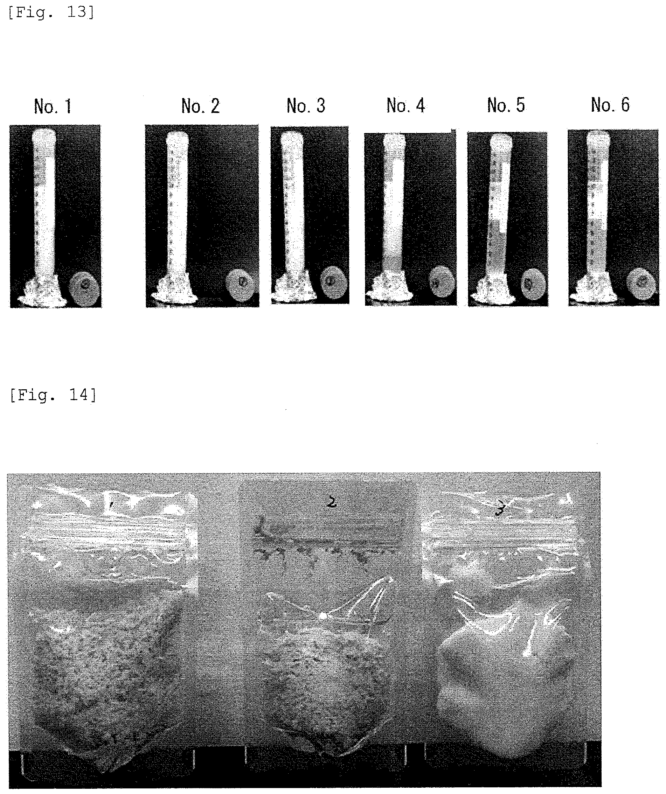

Fig. 13 is a photograph of each mixture obtained in Experimental Example 7 after emulsification. From the left, the photographs of samples No. 1, No. 2, No. 3, No. 4, No. 5, and No. 6 are shown.

[Fig. 14]

Fig. 14 is a photograph showing the emulsified state of each mayonnaise obtained in Experimental Example 8 when it was frozen overnight at -20°C and then left at ordinary temperature for 1 hr. From the left, the photographs of samples No. 1, No. 2, and No. 3 are shown.

[Fig. 15]

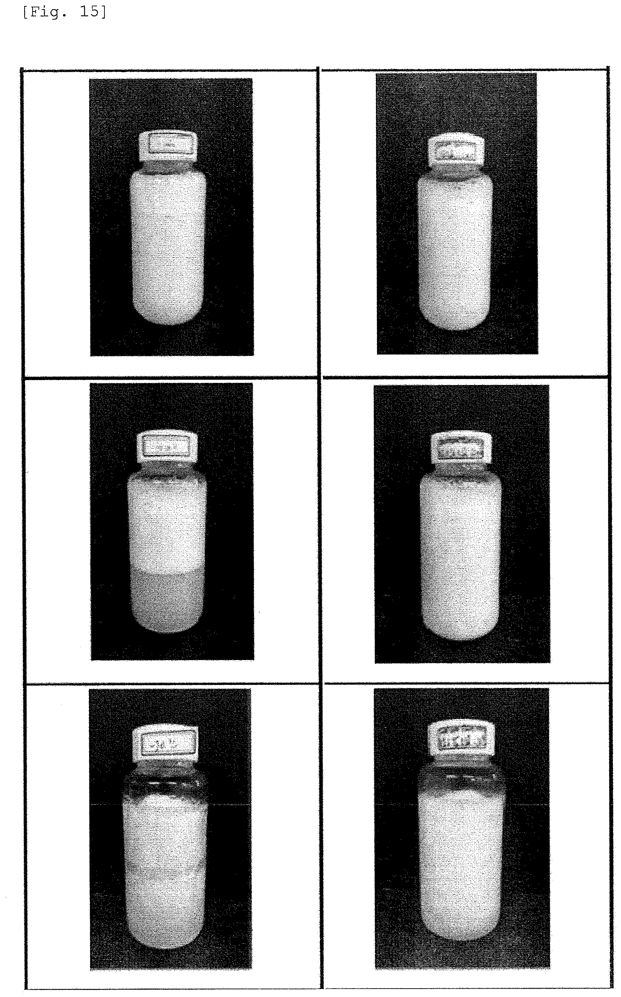

Fig. 15 is a photograph showing the appearance of each mixture obtained in Experimental Example 10 immediately after mixing (top row), after 60 min (middle row), and after freezing overnight at -20°C and thawing (bottom row). In addition, the left column is a photograph when a protein solution obtained according to the method described in Example 8 was used, and the right column is a photograph when the fluid gel prepared in Example 8 was used.

[Description of Embodiments]

1. Production method of fluid gel

The present invention provides a method for producing a fluid gel, including stirring a solution containing a protein and transglutaminase (hereinafter to be referred to as "the production method of the present invention").

In the present specification, the "fluid gel" is also called "liquid gel", "fluid-state gel", "fluid gel" and the like, and refers to an aggregate in which microgels are dispersed. Fluid gels have fluidity as a whole while gelling in the microgel parts, and behave differently from general gels which gelatinize as a whole, lose fluidity, and behave like a solid. In the present invention, the microgel is a microgel whose dispersoid is a protein treated with transglutaminase. The particle size of the microgel is not particularly limited. For example, the proportion of gel particles that pass through a 10,000 $\mu$m sieve is preferably not less than 50 wt%, more preferably not less than 70 wt%, further preferably not less than 90 wt%, of the total amount of the fluid gel.

The particle size of the fluid gel obtained by the production method of the present invention is preferably 0.1 $\mu$m to 1,000 $\mu$m as the median diameter (D50 particle size; hereinafter also referred to as "D50 particle size"), more preferably 0.1 $\mu$m to 500 $\mu$m, further preferably 0.1 $\mu$m to 200 $\mu$m. The median diameter can be measured, for example, using a laser diffraction·scattering particle size distribution measuring device LA-960 (Horiba, Ltd.).

As the dispersion medium constituting the fluid gel, water suitable for use in food production, such as purified water, distilled water, deionized water, tap water, and the like is preferred. Also, buffer solutions such as acetate buffer, phosphate buffer, and aqueous solutions suitable for use in food production, such as an aqueous sodium carbonate solution, can be used. The content of these dispersion media in the fluid gel is preferably 0.1 wt% to 30 wt%, more preferably 0.5 wt% to 20 wt%, and further preferably 1 wt% to 15 wt%.

Proteins include plant proteins, animal proteins, and mixtures of these, and the like.

Plant proteins are proteins derived from plants or compositions containing them, and also include those in which the protein content has been increased by processing plants. Specific examples of the plant protein include soy protein, wheat protein, green pea protein, mung bean protein, broad bean protein, green soy protein, rape seed protein, chia seed protein, corn protein, rice protein, buckwheat protein, sweet potato protein, asparagus protein, broccoli protein, avocado protein, oat protein, almond protein and the like, and soy protein is preferred.

More specifically, the plant protein is not particularly limited as long as it contains a protein derived from a plant. For example, the soy protein is not particularly limited as long as it contains a protein derived from soybeans, and refers to proteins that can be obtained by processing soybeans, such as concentrated soy protein, isolated soy protein, fibrous soy protein, extracted soy protein, and the like. Specific examples include, but are not limited to, "New FUJIPRO SEH" (Fuji Oil Co., Ltd.).

Operations such as plant processing, plant protein separation, and the like can be performed by a conventional method, for example, operations such as soybean processing, soy protein separation, and the like, and can be performed according to the methods described in Journal of the Japan Oil Chemists' Society, Vol. 19, No. 8, p. 826 (1970), Journal of the Japan Oil Chemists' Society, Vol. 28, No. 10, p. 781 (1979), and the like, or methods similar thereto.

As the plant protein, one type of plant protein may be used, or two or more types of plant proteins may be used.

Animal protein is a protein derived from an animal or a composition containing the same, and specific examples include milk protein (whey protein, casein protein, etc.), egg white protein, meat protein, fish protein, etc., of which milk protein or egg white protein is preferred, and casein protein is particularly preferred.

More specifically, the animal protein is not particularly limited as long as it contains a protein derived from an animal. For example, casein protein is not particularly limited as long as it contains a protein fraction obtained by removing whey and milk fat from raw milk, and refers to proteins such as micellar casein and sodium caseinate.

The animal protein may be prepared by oneself or may be a commercially available product such as "Sodium Caseinate 180" (Fonterra Japan K.K.) and the like.

As the animal protein, one type of animal protein may be used, or two or more types of animal proteins may be used.

Transglutaminase (TG) is an enzyme that has the activity of catalyzing an acyl transfer reaction in which a glutamine residue in a protein or peptide is used as a donor and a lysine residue is used as a receptor, and transglutaminase of various origins is known, such as those derived from mammals, those derived from fish, and those derived from microorganisms. The origin of the transglutaminase used in the present invention is not particularly limited as long as it has the aforementioned activity, and transglutaminase of any origin may be used, and recombinant enzymes may also be used. The transglutaminase used in the present invention may be a commercially available product, and specific examples include, but are not limited to, microorganism-derived transglutaminase commercially available under the trade names "Activa (registered trademark) TG-AK" (Ajinomoto Co., Inc.), "Activa (registered trademark) TG-S" (Ajinomoto Co., Inc.), "Activa TG (registered trademark)" (Ajinomoto Co., Inc.), and the like.

The amount of transglutaminase used in the production method of the present invention is not particularly limited, and may vary depending on the production conditions. It is preferably an amount that is 0.001 U (unit) to 1,000 U, more preferably an amount that is 0.01 U to 500 U, and further preferably an amount that is 0.1 U to 100 U, per 1 g of protein.

In the present invention, the activity unit of transglutaminase is measured and defined as follows. That is, transglutaminase is allowed to act in a reaction system in which benzyloxycarbonyl-L-glutamylglycine and hydroxylamine are used as substrates in Tris buffer at 37°C and pH 6.0, and the hydroxamic acid produced is allowed to form an iron complex in the presence of trichloroacetic acid. The absorbance at 525 nm is then measured, and the amount of hydroxamic acid is calculated using a calibration curve, and the amount of enzyme required to produce 1 $\mu$mol of hydroxamic acid per minute is defined as 1 unit (1 U) (see JP S64-27471 A).

As solvents for the solution, water suitable for use in food production, such as purified water, distilled water, deionized water, tap water, and the like is preferably used. Buffer solutions such as acetate buffer and phosphate buffer, and aqueous solutions suitable for use in food production, such as an aqueous sodium carbonate solution, can also be used.

The concentration of the protein in the solution is preferably 0.1 wt% to 40 wt%, more preferably 0.5 wt% to 30 wt%, and further preferably 1 wt% to 20 wt%.

The temperature at which the solution containing the mixture of protein and transglutaminase is stirred is preferably a temperature at which the protein does not denature, and depends on other conditions, but is preferably 0°C to 60°C, more preferably 10°C to 60°C, further preferably 20°C to 50°C. The stirring speed when the solution is stirred depends on other conditions. For example, 100 rpm to 30,000 rpm is preferred, 150 rpm to 20,000 rpm is more preferred, and 200 rpm to 15,000 rpm is further preferred. In one embodiment, 1,000 rpm to 30,000 rpm is more preferred and 2,000 rpm to 20,000 rpm is further preferred. Depending on other conditions, for example, a stirring speed of 100 rpm or more is preferred because it is less likely to cause the entire solution to gelatinize as it is. While the stirring time when the solution is stirred depends on other conditions, it is preferably 1 minute to 12 hours, more preferably 5 minutes to 6 hours, and further preferably 10 minutes to 3 hours.

In one embodiment, the production method of the present invention can be performed by stirring the solution containing the protein while adding transglutaminase.

In the production method of the present invention, components other than the protein and transglutaminase may be added as long as they do not impair the effects of the present invention. Examples of other components include other enzymes such as glucose oxidase and the like, alkaline agents (pH adjusters) such as sodium carbonate, potassium carbonate, calcined calcium, and the like, redox agents such as sodium ascorbate, glutathione, cysteine, and the like, and the like. As the other components, one type of component may be used, or two or more types of components may be used in combination.

In the production method of the present invention, the timing of adding the above-mentioned other components to the solution containing the protein and transglutaminase is not particularly limited.

In the production method of the present invention, the amount of other components to be used is not particularly limited, but is preferably an amount that is not more than 10 wt%, more preferably an amount that is not more than 5 wt%, and further preferably an amount that is not more than 1 wt%, in the solution containing the protein and transglutaminase.

2-1. Fluid gel 1

The present invention also provides a fluid gel containing a protein treated with transglutaminase, and having a median diameter of 0.1 $\mu$m to 1000 $\mu$m.

Transglutaminase, protein, fluid gel, and median diameter are as explained above in the production method of the present invention. The method of making transglutaminase act on protein is not particularly limited. An example is a method of stirring a solution containing a mixture of protein and transglutaminase as explained above in the production method of the present invention.

2-2. Fluid gel 2

The present invention also provides a fluid gel containing a protein treated with transglutaminase and satisfying one or more of the following properties at 25°C (hereinafter referred to as the "fluid gel of the present invention"):

   (1) coordination number in an angular frequency range of 0.1 to 100 rad/sec: 0.1 to 20
   (2) gel strength in an angular frequency range of 0.1 to 100 rad/sec: 0.1 Pas$^{1/z}$ to 100 Pas$^{1/z}$
   (3) viscosity coefficient in a shear rate range of 1 to 100/sec: 0.1 Pas$^n$ to 100 Pas$^n$.

In one embodiment, the present invention provides a fluid gel containing a protein treated with transglutaminase, which satisfies two or more of the above-mentioned properties (1) to (3) at 25°C.

In one embodiment, the present invention provides a fluid gel containing a protein treated with transglutaminase, which satisfies all of the above-mentioned properties (1) to (3) at 25°C.

Transglutaminase, protein, and fluid gel are as explained above in the production method of the present invention. The method of making transglutaminase act on protein is not particularly limited. An example is a method of stirring a solution containing a mixture of protein and transglutaminase as explained above in the production method of the present invention.

The complex modulus refers to the complex expression of the elastic modulus with respect to oscillatory stress and strain in linear viscoelasticity (Chemical Dictionary, 2nd Edition, Morikita Publishing (2009)). The complex modulus can be measured, for example, using a rheometer such as the Stress Controlled Rheometer MCR501 (Anton Paar Japan Co., Ltd.).

In the fluid gel of the present invention, the complex modulus at an angular frequency of 1 rad/sec is preferably 5 Pa to 5,000 Pa, more preferably 10 Pa to 2,000 Pa.

The coordination number is information relating to the spatial distribution of virtual flow unit lattices in the system (RHEOLOGY TORONKAI KOEN YOSHISHU (Proceedings of the Rheology Symposium), vol.: 66th, p. 322-323 (October 17, 2018)), and the gel strength indicates the strength of the mechanical interaction between the above-mentioned flow unit lattices (RHEOLOGY TORONKAI KOEN YOSHISHU (Proceedings of the Rheology Symposium), vol.: 66th, p. 322-323 (October 17, 2018)). Both the coordination number and gel strength can be derived from the angular frequency dependence data of the above-mentioned complex modulus using a weak-gel model (J Biorheol, 32 (1), 9-14 (2018), Rheologica Acta, 40, 120-127 (2001), etc.). That is, first, the angular frequency dependency data in the angular frequency range of 0.1 to 100 rad/sec is plotted on a graph with the complex modulus (Pa) on the vertical axis and the angular frequency (rad/sec) on the horizontal axis, and an approximate line is drawn on the graph by the least squares method, and when the vertical intercept of the approximate line at an angular frequency of 1 rad/sec is b and the slope of the approximate line is a, the gel strength $A_f$(Pas$^{1/z}$) in the angular frequency range of 0.1 to 100 rad/sec is expressed as $10^b$, and the coordination number z is expressed as 1/a. In addition, the complex modulus, coordination number, gel strength, and angular frequency have the following relationship in the weak-gel model (Rheologica Acta, 40, 120-127 (2001)).

[numerical formula 1]

$$G^* = A_f \omega^{1/z}$$

(In the formula, G* represents the complex modulus (Pa), $A_f$ represents the gel strength (Pas$^{1/z}$), $\omega$ represents the angular frequency (rad/sec), and z represents the coordination number).

In the fluid gel of the present invention, the coordination number in the angular frequency range of 0.1 to 100 rad/sec is preferably 0.1 to 15, more preferably 0.1 to 10. In addition, the gel strength in the angular frequency range of 0.1 to 100 rad/sec is preferably 0.1 Pas$^{1/z}$ to 80Pas$^{1/z}$, more preferably 0.1Pas$^{1/z}$ to 60Pas$^{1/z}$.

The shear stress can also be measured using a rheometer such as the Stress Controlled Rheometer MCR501 (Anton Paar Japan Co., Ltd.), and the viscosity coefficient can be analyzed using the shear stress and the shear rate set during measurement with the rheometer, along with the H-B (Herschel-Bulkley) index and yield stress, using the analysis software Universal Software US200 (Paar Physica) according to the following Herschel-Bulkley equation (Kolloid-Z, 39, 291 (1926)), an empirical equation that describes non-Newtonian flow.

[numerical formula 2]

$$\sigma = k\dot{\gamma}^n + \sigma_y$$

(In the formula, $\sigma$ represents shear stress (Pa), k represents viscosity coefficient (Pas$^n$), $\gamma$ represents shear rate (1/sec), n represents H-B index (no unit), and $\sigma_y$ represents yield stress (Pa)).

In the fluid gel of the present invention, the shear stress at a shear rate of 10/sec is preferably 2 Pa to 200 Pa, more preferably 5 Pa to 100 Pa. Also, the viscosity coefficient at a shear rate range of 1 to 100/sec is preferably 0.1 Pas$^n$ to 80 Pas$^n$, more preferably 0.1 Pas$^n$ to 60 Pas$^n$.

The average particle size of the fluid gel of the present invention is preferably 0.1 $\mu$m to 1,000 $\mu$m, more preferably 0.1 $\mu$m to 500 $\mu$m, and further preferably 0.1 $\mu$m to 200 $\mu$m. The average particle size can be measured, for example, using a laser diffraction-scattering particle size distribution measuring device LA-960 (Horiba, Ltd.).

The fluid gel of the present invention can be produced, for example, by the above-mentioned production method of the present invention.

In one embodiment, the present invention provides a fluid gel that can be obtained by stirring a solution containing a protein and transglutaminase.

In one embodiment, the present invention also provides a fluid gel that contains a protein treated with transglutaminase, the fluid gel having an average particle size of 0.1 μm to 1,000 um. This fluid gel can also be produced, for example, by the above-mentioned production method of the present invention.

3. Emulsifier containing fluid gel

The present invention also provides an emulsifier containing the fluid gel of the present invention (hereinafter referred to as the "emulsifier of the present invention").

The emulsifier of the present invention may be the fluid gel of the present invention used alone, or may contain other components as long as the effect of the present invention is not impaired. Examples of other components include other emulsifiers such as sucrose fatty acid ester, glycerol fatty acid ester, sorbitan fatty acid ester, sodium caseinate, lecithin, saponin, and the like, thickeners such as xanthan gum, gellan gum, gum arabic, carrageenan and the like, proteins such as egg white and the like, carbohydrates such as glucose, lactose, and the like, fats and oils such as soybean oil and the like, sweeteners such as aspartame, acesulfame potassium, and the like, flavoring materials such as sodium chloride, sodium L-glutamate, and the like, colorants such as annatto dye, turmeric dye, gardenia dye, and the like, flavors such as ethyl acetoacetate, orange, lavender and the like, preservatives such as benzoic acid, sodium benzoate, sorbic acid, and the like, and the like.

The content of the fluid gel of the present invention in the emulsifier of the present invention is not particularly limited. It is preferably 0.01% to 100 wt%, more preferably 1 wt% to 100 wt%, 10 wt% to 100 wt%, 30 wt% to 100 wt%, or 50 wt% to 100 wt%, further preferably 70 wt% to 100 wt%, or 80 wt% to 100 wt%, particularly preferably 90 wt% to 100 wt%.

The content of the above-mentioned other components in the emulsifier of the present invention is not particularly limited. It is preferably 0.0001 wt% to 30 wt%, more preferably 0.001 wt% to 20 wt%, further preferably 0.01 wt% to 10 wt%, in total.

A method for producing the emulsifier of the present invention is now described.

The emulsifier of the present invention may be produced by using the fluid gel of the present invention as it is, or, for example, by adding the above-mentioned other components to the fluid gel of the present invention and mixing them by a general method.

The emulsifier of the present invention can be added, for example, to emulsified foods, cosmetics, and the like.

The emulsified foods to which the emulsifier of the present invention is added are preferably oil-in-water emulsified foods. Examples of the oil-in-water emulsified food include yoghurt, mayonnaise, whipped cream, ice cream, custard cream, cafe au lait, coffee cream, coffee drinks, and the like.

The content of the emulsifier in the emulsified food is not particularly limited, but is preferably 0.01 wt% to 30 wt%, more preferably 0.05 wt% to 20 wt%, and further preferably 0.1 wt% to 10 wt%. The timing of adding the emulsifier of the present invention to an emulsified food is not particularly limited. For example, it may be mixed together with the other ingredients of the emulsified food, or it may be added after mixing the other ingredients. The emulsifier of the present invention may also be added to an emulsified food in combination with other emulsifiers such as sucrose fatty acid ester, glycerol fatty acid ester, sorbitan fatty acid ester, sodium caseinate, lecithin, saponin, and the like.

Examples of the cosmetics to which the emulsifier of the present invention is added include basic cosmetics such as emulsion, cream, lotion, facial cleanser, and the like, hair cosmetics such as shampoo, rinse, treatment and the like, makeup cosmetics such as liquid foundation and the like, and the like.

The content of the emulsifier in the cosmetics is not particularly limited, but is preferably 0.01 wt% to 80 wt%, more preferably 0.05 wt% to 60 wt%, further preferably 0.1 wt% to 40 wt%. The timing of the addition of the emulsifier of the present invention to a cosmetic is not particularly limited. For example, the emulsifier may be mixed together with the other ingredients of the cosmetic, or may be added after mixing the other ingredients. The emulsifier of the present invention may also be added to a cosmetic in combination with other emulsifiers such as sodium caseinate, saponin, and the like.

4. Semi-solid food containing fluid gel

The present invention also provides a semi-solid food containing the fluid gel of the present invention (hereinafter to be referred to as "the semi-solid food of the present invention").

Semi-solid foods include yoghurt, mayonnaise, whipped cream, cafe au lait, dressings, sauces, and the like, and yoghurt, mayonnaise, whipped cream, and cafe au lait are preferred. Here, semi-solid foods include alternative semi-solid foods; for example, mayonnaise includes conventional mayonnaise, as well as alternative mayonnaise such as low-fat mayonnaise and mayonnaise using plant protein instead of animal protein; whipped cream includes whipped cream using milk fat (raw milk) (hereinafter referred to as "milk fat"), which is animal fat with high fat content, as well as alternative whipped cream such as whipped cream using milk fat with low fat content and whipped cream using vegetable fat (hereinafter referred to as "vegetable fat").

Low-fat mayonnaise, mayonnaise using plant protein instead of animal protein, and the like have a thin viscosity, and therefore, a thickener such as xanthan gum is generally added. This results in a sticky texture, and whipped cream using milk fat with low fat content and whipped cream using vegetable fat have low foaming property. Thus, until now it has been difficult to achieve the properties of conventional mayonnaise and whipped cream using milk fat with high fat content, respectively. Therefore, alternative semi-solid foods such as alternative mayonnaise and whipped cream in place of low-fat mayonnaise, which is prone to problems in terms of property, mayonnaise using plant protein instead of animal protein, whipped cream using milk fat with low fat content, whipped cream using vegetable fat, and the like are more preferred as semi-solid foods.

A method for producing the semi-solid food of the present invention is now described.

The semi-solid food of the present invention can be produced, for example, by adding the fluid gel of the present invention to the raw materials of the food during the production process of the semi-solid food and mixing them by a general method. The raw materials of the food refer to food materials for producing the food, and are not particularly limited as long as the food can be produced, and anything that is generally used as a raw material for the food can be used. The raw materials of the food also include intermediate products of the food at any stage during the production process of the food.

5. Production method of semi-solid food and method for improving property of semi-solid food

The present invention also provides a method for producing a semi-solid food and a method for improving the property of a semi-solid food, which is characterized by mixing the fluid gel of the present invention with the raw materials of the semi-solid food.

The semi-solid food and the raw materials of the food are each the same as those described above for the semi-solid food of the present invention.

The method for producing a semi-solid food and the method for improving the property of the present invention can be performed, for example, by adding the fluid gel of the present invention to the raw materials of the semi-solid food during the production process of the semi-solid food and mixing them by a general method.

For example, when the semi-solid food is mayonnaise, the property of the mayonnaise is improved (stickiness-free viscosity and a rich taste, good melt in mouth and the like are obtained), when the semi-solid food is whipped cream, the property of the whipped cream is improved (foaming property is improved), and when the semi-solid food is cafe au lait, the property of the cafe au lait is improved (rich taste similar to that of oil and fat, and the mid- to late-stage thickness are imparted).

The present invention is now explained in detail with reference to examples and the like, but the present invention is not limited to these examples.

[Example]

Preparation Example 1 (production of fluid gel; alkali condition)

Soy protein is added to a mixture of 185.9 mL of distilled water and 2.1 mL of 10 mM $Na_2CO_3$ aqueous solution (pH 10.5), stirred and dissolved in a homomixer at 40°C and 15,000 rpm for 1 hr, and the mixture is defoamed in a centrifuge at 2,000 rpm for 5 min to obtain a soy protein solution. Transglutaminase is added to the resulting solution, and the mixture is stirred in a homomixer at 40°C and 5,000 rpm for 30 min. The resulting solution is heated in a microwave oven (900W, 30 sec), and then heated in an oil bath at 75°C or higher for 5 min to inactivate the transglutaminase. The solution is then defoamed in a centrifuge at 2,000 rpm for 10 min to obtain a fluid gel.

Preparation Example 2 (production of fluid gel; neutral condition)

Soy protein is added to 188.0 mL of distilled water, and 4.0 mL of ethanol is added to preliminarily disperse the soy protein. It is then stirred and dissolved in a homomixer at 60°C and 15,000 rpm for 1 hr, and defoamed in a centrifuge at 2,000 rpm for 5 min to obtain a soy protein solution. Transglutaminase is added to the resulting solution, and the mixture is stirred in a homomixer at 40°C and 5,000 rpm for 30 min. The resulting solution is heated in a microwave oven (900W, 30 sec), and then heated in an oil bath at 75°C or higher for 5 min to inactivate the transglutaminase. The solution is then defoamed in a centrifuge at 2,000 rpm for 10 min to obtain a fluid gel.

Example 1 (production of fluid gel; alkali condition)

Following the method described in Preparation Example 1, 5 wt%, 6 wt%, and 7 wt% soy protein solutions were obtained from soy protein "New FUJIPRO SEH" (Fuji Oil Co., Ltd.), distilled water, and 10 mM $Na_2CO_3$ aqueous solution (pH 10.5). Transglutaminase "Activa (registered trademark) TG" was added to each of the obtained solutions so that the concentration thereof was 12.2 U/g, and each was stirred in a homomixer for 30 min at 40°C and 300 rpm instead of 5,000 rpm. The obtained solutions were heated in a microwave oven (900 W, 30 sec), and then heated in an oil bath at 75°C or higher for 5 min to inactivate the transglutaminase. The solutions were then defoamed in a centrifuge at 2,000 rpm for 10 min. As a control, the same procedure was performed without adding transglutaminase.

As a result, when transglutaminase was added, a shape resembling a fluid gel particle was visually observed regardless of the concentration of soy protein solution used, but when transglutaminase was not added, a shape resembling a fluid gel particle was not visually observed regardless of the concentration of soy protein solution used.

Example 2 (production of fluid gel; alkali condition)

Following the method described in Preparation Example 1, 5 wt% soy protein solution was obtained from soy protein "New FUJIPRO SEH" (Fuji Oil Co., Ltd.), distilled water, and 10 mM $Na_2CO_3$ aqueous solution (pH 10.5). Transglutaminase "Activa (registered trademark) TG" was added to the obtained solution so that the concentration thereof was 1.22 U/g, 3.66 U/g or 6.10 U/g, and each was stirred in a homomixer for 30 min at 40°C and 5,000 rpm. The obtained solutions were heated in a microwave oven (900 W, 30 sec), and then heated in an oil bath at 75°C or higher for 5 min to inactivate the transglutaminase. The solutions were then defoamed in a centrifuge at 2,000 rpm for 10 min.

Photographs of the resulting mixtures are shown in Fig. 1, respectively. As shown in Fig. 1, when transglutaminase was added to 1.22 U/g and 3.66 U/g, a transparent fluid gel was obtained. On the other hand, when transglutaminase was added to 6.10 U/g, the viscosity rose sharply and the entire mixture gelled.

Example 3 (production of fluid gel; neutral condition)

Following the method described in Preparation Example 2, 5 wt% soy protein solution was obtained from soy protein "New FUJIPRO SEH" (Fuji Oil Co., Ltd.) and distilled water. Transglutaminase "Activa (registered trademark) TG" was added to the obtained solution so that the concentration thereof was 1.22 U/g, 3.66 U/g or 6.10 U/g, and each was stirred in a homomixer for 30 min at 40°C and 5,000 rpm. The obtained solutions were heated in a microwave oven (900 W, 30 sec), and then heated in an oil bath at 75°C or higher for 5 min to inactivate the transglutaminase. The solutions were then defoamed in a centrifuge at 2,000 rpm for 10 min.

Photographs of the resulting mixtures are shown in Fig. 2, respectively. As shown in Fig. 2, when transglutaminase was added to 1.22 U/g and 3.66 U/g, a transparent fluid gel was obtained. On the other hand, when transglutaminase was added to 6.10 U/g, the viscosity rose sharply and the entire mixture gelled.

Experimental Example 1 (Measurement of angular frequency dependence of complex modulus and coordination number)

For each fluid gel obtained when transglutaminase was added in Example 1, the angular frequency dependence of complex modulus (G*) was measured under the following conditions, and then the coordination number (z) in the angular frequency range of 0.1 to 100 rad/sec was derived.

- Measurement equipment: Stress Controlled Rheometer MCR501 (Anton Paar Japan Co., Ltd.)
- Measurement temperature: 25°C
- Jig: CP25, CP50, CC17
- Gap: 1.0 mm, 1.5 mm
- Angular frequency when measuring angular frequency dependence of complex modulus: 0.3 to 30 rad/sec
- Distortion when measuring angular frequency dependence of complex modulus: 1%, 3%

The coordination number (z) in the angular frequency range of 0.1 to 100 rad/sec was derived by plotting the angular frequency dependency data of the above-mentioned complex modulus (G*) (Pa) in the angular frequency range of 0.1 to 100 rad/sec on a graph with the complex modulus (Pa) on the vertical axis and the angular frequency (rad/sec) on the horizontal axis, drawing an approximation line by the least squares method, and calculating 1/a from the slope a of the approximation line.

The results thereof are shown in Fig. 3 and Fig. 4. As shown in Fig. 3 and Fig. 4, the complex modulus and coordination number increased as the protein concentration was increased. In other words, it can be seen that the more the protein concentration was increased, the more fluid gel particles were formed.

Experimental Example 2 (measurement of shear stress)

The shear stress was measured under the following conditions for each fluid gel obtained in Example 2 and Example 3.

- Measurement equipment: Stress Controlled Rheometer MCR501 (Anton Paar Japan Co., Ltd.)
- Measurement temperature: 25°C
- Jig: CP25, CP50, CC17
- Gap: 1.0 mm, 1.5 mm
- Shear rate during steady-flow viscosity measurement: 1 to 100/sec

The results thereof are shown in Fig. 5 and Fig. 6. As shown in Fig. 5 and Fig. 6, it can be seen that the higher the transglutaminase concentration, the higher the shear stress becomes, namely, the viscosity of the fluid gel increases more.

Experimental Example 3 (measurement of viscosity coefficient)

The viscosity coefficient was derived from the shear stress measured in Experimental Example 2 for each fluid gel obtained when transglutaminase "Activa (registered trademark) TG" was added to 1.22 U/g and 3.66 U/g in Example 2 (alkaline conditions) and Example 3 (neutral conditions).
In other words, the viscosity coefficient was calculated by analyzing the shear stress obtained in Experimental Example 2 and the shear rate (1/sec) set during measurement with the above-mentioned rheometer using the following Herschel-Bulkley equation using analysis software Universal Software US200 (Paar Physica).

[numerical formula 3]

$$\sigma = k\dot{\gamma}^{n} + \sigma_{y}$$

(In the formula, $\sigma$ represents shear stress (Pa), k represents viscosity coefficient ($Pas^{n}$), $\gamma$ represents shear rate (1/sec), n represents H-B index (no unit), and $\sigma_{y}$ represents yield stress (Pa)).
The results thereof are shown in Fig. 7 and Fig. 8. As shown in Fig. 7 and Fig. 8, it can be seen that the higher the transglutaminase concentration, the higher the viscosity coefficient becomes, namely, the viscosity of the fluid gel increases more.

Experimental Example 4 (measurement of gel strength)

The complex modulus (G*) of each fluid gel obtained when the transglutaminase "Activa (registered trademark) TG" was added to 1.22 U/g and 3.66 U/g in Example 2 was measured under the following conditions.

- Measurement equipment: Stress Controlled Rheometer MCR501 (Anton Paar Japan Co., Ltd.)
- Measurement temperature: 25°C
- Jig: CP25, CP50, CC17
- Gap: 1.0 mm, 1.5 mm
- Angular frequency when measuring angular frequency dependence of complex modulus: 0.3 to 30 rad/sec
- Distortion when measuring angular frequency dependence of complex modulus: 1%, 3%

Then, the gel strength $A_{f}$ ($Pas^{1/z}$) at an angular frequency of 1 rad/sec was derived by plotting the angular frequency dependency data of the above-mentioned complex modulus (G*) (Pa) in the angular frequency range of 0.1 to 100 rad/sec on a graph with the complex modulus (Pa) on the vertical axis and the angular frequency (rad/sec) on the horizontal axis, drawing an approximation line by the least squares method, and calculating $10^{b}$ from the vertical intercept b of the approximation line at an angular frequency of 1 rad/sec.
The results thereof are shown in Fig. 9. As shown in Fig. 9, it can be seen that the gel strength increases as the transglutaminase concentration increases.

Example 4 (preparation of mayonnaise)

Mayonnaise was prepared with the composition shown in Table 1.

[Table 1]

| proportion (%) | full fat mayonnaise (positive control) | half mayonnaise + thickener (negative control) | half mayonnaise + fluid gel | vegetable mayonnaise + fluid gel |
|---|---|---|---|---|
| egg-yolk | 13.2 | 13.2 | 13.2 | - |
| xanthan gum | - | 0.8 | - | - |
| vinegar | 1.4 | 5 | 5 | 4 |
| salt | 1.4 | 5 | 5 | 2.4 |
| MSG | 0.01 | 0.04 | 0.04 | 0.05 |
| granulated sugar | - | - | - | 2.5 |
| starch sirup | - | - | - | 3 |
| water | 11 | 39 | - | - |
| canola oil | 73 | 37 | 37 | 60 |
| soybean 6%fluid gel | - | - | 39.8 | 28 |
| total | 100.0 | 100.0 | 100.0 | 100.0 |

The soybean 6% fluid gel used was a fluid gel obtained from a 6 wt% soy protein solution prepared in the same manner as in Example 1. Half mayonnaise corresponds to low-fat mayonnaise.

Experimental Example 5 (measurement of dynamic viscoelasticity)

Dynamic viscoelasticity was measured for each mayonnaise obtained in Example 4, under the following conditions and using an ARES G-2 (manufactured by TA Instruments) as the measuring device. Specifically, about 2 ml of each sample was dropped directly under the jig, and after it reached the gap, the protruding sample was wiped off, and the measurement was performed.

- Distortion sweep: 0.1% to 1000%
- Frequency: 1 Hz
- Gap: 0.2 mm
- Jig: Parallel plate ($\varphi$50 mm)

G' (storage modulus (index of elastic property)), G" (loss modulus (index of viscous property)), and Tan $\delta$ (loss tangent (G'/G")) were measured at distortion rates of 0.3%, 1%, 5%, 10%, 20%, 50%, 100%, 200%, 400%, 500%, 800% and 1,000%, and the results were used to perform principal component analysis of the top parameters at each distortion rate to obtain a score plot.

The results thereof are shown in Fig. 10. As shown in Fig. 10, in the plot consisting of the first principal component and second principal component, the commercially available full-fat mayonnaise, the full-fat mayonnaise obtained in Example 4, the half mayonnaise + fluid gel, and the vegetable mayonnaise + fluid gel were arranged close to each other in the right direction from the point of origin. On the other hand, the commercially available half mayonnaise, which is a low-fat mayonnaise, was arranged leftward from the point of origin, and the half mayonnaise containing the thickener xanthan gum obtained in Example 4 was also arranged leftward from the point of origin. In addition, the commercially available vegetable mayonnaise was arranged upward from the point of origin. As described above, a map was obtained that was roughly divided into full-fat type, low-fat type, and vegetable type. Furthermore, although not shown, the relationship among the full-fat type, low-fat type, and vegetable type did not change when looking at the plot consisting of the first principal component and the third principal component and the plot consisting of the second principal component and the third principal component. In other words, it is considered that the addition of the fluid gel of the present invention to low-fat mayonnaise (half mayonnaise and vegetable mayonnaise) makes it possible to reproduce a texture like that of full-fat mayonnaise.

Example 5 (preparation of whipped cream)

A mug was filled with (1) milk fat with a low fat content (fat content 35%), (2) vegetable fat (fat content 41%), or (3) vegetable fat (fat content 41%) (about 100 ml), and the mug was immersed in a bowl of ice water. 7.5 g of sugar and, in the case of (3), 2% (2 g) of fluid gel obtained from a 5 wt% soy protein solution prepared in the same manner as in Example 1 were added. The mixture was whipped using a Bamix until the corners stood up, and then leveled with and placed in a plastic cup to prepare whipped cream, which was then weighed.

The results thereof are shown in Table 2 and Fig. 11.

[Table 2]

| sample name | plastic cup-leveled weight (g) | overrun (%) | time required for whipping |
|---|---|---|---|
| milk fat with low fat content (fat content 35%) | 53.63 | 24.1 | 60 sec |
| vegetable fat (fat content 41%) | 55.95 | 20.9 | 5 min |
| vegetable fat (fat content 41%)+fluid gel 2% (2 g) | 51.72 | 26.8 | 5 min |

As shown in Table 2 and Fig. 11, as compared with (1) the whipped cream prepared from milk fat with a low fat content, (2) the whipped cream prepared from vegetable fat had a lower overrun value, but (3) the whipped cream prepared from a mixture in which a fluid gel obtained from a 5 wt% soy protein solution was added to vegetable fat had a higher overrun value than (1) the whipped cream prepared from milk fat with a low fat content. In other words, it is clear that the addition of the fluid gel of the present invention improves the foaming property of the obtained whipped cream.

Experimental Example 6 (measurement of dynamic viscoelasticity)

Dynamic viscoelasticity was measured for each whipped cream obtained in Example 5, under the following conditions and using an ARES G-2 (manufactured by TA Instruments) as the measuring device. Specifically, about 2 ml of each sample was dropped directly under the jig, and after it reached the gap, the protruding sample was wiped off, the measurement was performed, and G' (storage modulus (index of elastic property)) was calculated.

- Distortion sweep: 0.1% to 1000%
- Frequency: 1 Hz
- Gap: 0.2 mm
- Jig: Parallel plate ($\varphi$50 mm)

The results thereof are shown in Fig. 12. As shown in Fig. 12, the storage modulus when the distortion increased was higher in (2) the whipped cream prepared from vegetable fat, compared to (1) the whipped cream prepared from milk fat with a low fat content, but in (3) the whipped cream prepared from a mixture in which a fluid gel obtained from a 5 wt% soy protein solution was added to vegetable fat, the storage modulus decreased to the same level as (1) the whipped cream prepared from milk fat with a low fat content. In other words, it is clear that the addition of the fluid gel of the present invention improves the melt in mouth of the obtained whipped cream.

Experimental Example 7 (evaluation of emulsifying property)

7 g of a fluid gel sample or a comparison sample and 3.5 g of canola oil were added to a 15 ml Eppendorf tube, and emulsified by stirring at 5,000 rpm for 1 min with a homogenizer. Thereafter, the appearance was measured 1 hr later. As fluid gel samples, fluid gels obtained from 5 wt%, 6 wt%, and 7 wt% soy protein solutions (samples No. 1, No. 2, and No. 3, respectively) prepared in the same manner as in Example 1 were used, and as comparison samples, samples No. 4, No. 5, and No. 6 with compositions shown in Table 3 were used (only sample No. 4 was over 7 g).

[Table 3]

| sample name | soybean (g) | transglutaminase (U) | lecithin (g) | $Na_2CO_3$ (g) | water (g) |
|---|---|---|---|---|---|
| No. 4 | 0.42 | 85.4 | - | 0.0658 | 6.58 |
| No. 5 | - | - | 0.035 | - | 6.965 |

(continued)

| sample name | soybean (g) | transglutaminase (U) | lecithin (g) | Na$_2$CO$_3$ (g) | water (g) |
|---|---|---|---|---|---|
| No. 6 | - | - | 0.105 | - | 6.895 |

A photograph of the appearance after 1 hr is shown in Fig. 13. As shown in Fig. 13, when sample No. 4, which was simply a mixture of soy protein and transglutaminase, and sample No. 5 and sample No. 6, which contained lecithin, which is generally known as an emulsifier, were used, layer separation occurred, but when the fluid gels obtained by the production method of the present invention (samples No. 1, No. 2, and No. 3) were used, layer separation was suppressed, confirming the high emulsifying property of the fluid gels.

Example 6 (preparation of mayonnaise)

Mayonnaise was prepared with the composition shown in Table 4.

[Table 4]

| proportion (%) | sample name | | |
|---|---|---|---|
| | No. 1 | No. 2 | No. 3 |
| egg-yolk | 13.2 | 13.2 | 13.2 |
| vinegar | 1.4 | 1.4 | 1.4 |
| salt | 1.4 | 1.4 | 1.4 |
| MSG | 0.01 | 0.01 | 0.01 |
| water | 11 | - | - |
| canola oil | 73 | 73 | 73 |
| soybean 6% fluid gel 300 rpm | - | 11 | - |
| soybean 6% fluid gel 5,000 rpm | - | - | 11 |
| total | 100.0 | 100.0 | 100.0 |

The fluid gel used was "soybean 6% fluid gel 300 rpm", which was obtained by adding transglutaminase "Activa (registered trademark) TG" to the soy protein solution to a concentration of 3.66 U/g and stirring at 300 rpm instead of 5,000 rpm in Example 2, or "soybean 6% fluid gel 5,000 rpm", which was obtained by adding transglutaminase "Activa (registered trademark) TG" to the soy protein solution to a concentration of 3.66 U/g in Example 2.

Experimental Example 8 (evaluation of emulsifying property)

Each mayonnaise prepared in Example 6 was frozen at -20°C overnight and then left at room temperature for 1 hr, and the emulsified state was observed.
The results thereof are shown in Fig. 14. As shown in Fig. 14, layer separation was observed in mayonnaise without fluid gel and mayonnaise containing fluid gel stirred at 300 rpm, whereas it was confirmed that mayonnaise containing fluid gel stirred at 5,000 rpm maintained a good emulsified state even after thawing.

Example 7 (preparation of cafe au lait)

Cafe au lait was prepared with the composition shown in Table 5.

[Table 5]

| proportion (g) | sample name | | |
|---|---|---|---|
| | No.1 | No. 2 | No. 3 |
| ICP | 4.5 | - | - |
| reduced-fat ICP (20% fat reduction) | - | 4.5 | 4.5 |

(continued)

| proportion (g) | sample name | | |
|---|---|---|---|
| | No.1 | No. 2 | No. 3 |
| fluid gel | - | | 0.225 |
| premix | 2.73 | 2.73 | 2.73 |
| hot water | 180 | 180 | 180 |
| mid- to late-stage thickness evaluation | - | × × | ○ |

Note that ICP refers to instant creaming powder that is added to luxury beverages such as coffee drinks as a substitute for cream, and Marim (registered trademark) (Ajinomoto AGF Co., Ltd.) was used. For the reduced-fat ICP, Marim (registered trademark) (Ajinomoto AGF Co., Ltd.) with 20% less fat was used.

The premix was prepared from instant coffee powder, sodium bicarbonate, and granulated sugar according to the method described in JP-A-2005-144600.

The fluid gel used was the one obtained in Example 2 by adding transglutaminase "Activa (registered trademark) TG" to the soy protein solution to 3.66 U/g.

Experimental Example 9 (Evaluation of mid- to late-stage thickness)

The mid- to late-stage thickness of each cafe au lait prepared in Example 7 was evaluated by four panelists skilled in evaluation through discussion, using the mid- to late-stage thickness of cafe au lait measured using ICP as the standard and according to the following evaluation scale. The "mid- to late-stage thickness" refers mainly to the thickness derived from fats and oils and milk, and indicates a sensation of intensification of the flavor without changing the balance.

⊙: greatly exceeds the thickness in the mid- to late-stage of cafe au lait using ICP
○○: greater than the thickness in the mid- to late-stage of cafe au lait using ICP
O: equivalent to the thickness in the mid- to late-stage of cafe au lait using ICP
x; less than the thickness in the mid- to late-stage of cafe au lait using ICP
xx; greatly below the thickness in the mid- to late-stage of cafe au lait using ICP

The results thereof are shown in Table 5. As shown in Table 5, in the cafe au lait using reduced-fat ICP, the thickness in the mid- to late-stage was much less than that using ICP, and it was watery, thin, less thick and rich, and less milky; however, in the cafe au lait using reduced-fat ICP and fluid gel, the thickness in the mid- to late-stage was equivalent to that using ICP.

Example 8 (production of fluid gel using milk protein)

Milk protein "Sodium Casainate 180" (Fonterra Japan Co., Ltd.) was added to water at 60°C so that the protein concentration was 8 wt%, and mixed using a homogenizer at 10,000 rpm for 1 min, and then at 5,000 rpm for 59 min to obtain 500 mL of a protein solution. After the temperature of the obtained solution was lowered to 40°C, transglutaminase "Activa (registered trademark) TG" was added to 300 mL of the solution so that the concentration thereof was 3.66 U/g, and the mixture was stirred in a homomixer at 40°C and 1,0000 rpm for 30 min. The obtained solution was heated in an oil bath at 75°C or higher for 5 min or more to inactivate the transglutaminase. The mixture was then defoamed in a centrifuge at 2,000 rpm for 10 min to obtain a fluid gel.

Experimental Example 10 (evaluation of emulsifying property)

14 g of the fluid gel prepared in Example 8, 126 g of water, and 60 g of canola oil were mixed for 5 min at 5,000 rpm using a homogenizer. As a control, the same procedure was performed using a protein solution obtained according to the method described in Example 8 instead of the fluid gel prepared in Example 8.

The appearance of each solution obtained was observed immediately after mixing, after 60 min, and after freezing overnight at -20°C and thawing.

The results thereof are shown in Fig. 15. As shown in Fig. 15, when the protein solution obtained according to the method described in Example 8 was used, layers were separated immediately after mixing, but when the fluid gel prepared in Example 8 was used, no layer separation was observed until after freezing and thawing.

Experimental Example 11 (particles size measurement 1)

The particle sizes of each fluid gel prepared using a 6 wt% soy protein solution instead of the 5 wt% soy protein solution and stirring at 10,000 rpm instead of 5,000 rpm in Example 2, and the protein solution prepared using a 6 wt% soy protein solution instead of the 5 wt% soy protein solution and stirring at 10,000 rpm instead of 5,000 rpm without adding transglutaminase "Activa (registered trademark) TG" in Example 2 were measured using a laser diffraction-scattering particle size distribution measuring device LA-960 (Horiba, Ltd.).

The results thereof (D50 particle size) are shown in Table 6.

[Table 6]

| amount of TG added (U/g) | D50 ($\mu$m) |
|---|---|
| 0.00 | 62 |
| 1.22 | 82 |
| 3.66 | 78 |
| 6.10 | 66 |

In addition, the particle size was measured using a laser diffraction-scattering particle size distribution measuring device LA-960 (HORIBA, Ltd.) for a fluid gel prepared using a 6 wt% soy protein solution instead of the 5 wt% soy protein solution in Example 2, and a fluid gel prepared using a 6 wt% soy protein solution instead of the 5 wt% soy protein solution and stirring at 300 rpm instead of 5,000 rpm in Example 2.

As a result, the D50 particle size was approximately 200 $\mu$m when stirred at 5,000 rpm, and the D50 particle size was about 1,000 $\mu$m when stirred at 300 rpm.

Experimental Example 12 (particles size measurement 2)

The particle sizes of the fluid gel prepared in Example 8, the fluid gel prepared by adding transglutaminase "Activa (registered trademark) TG" to 1.22 U/g, 2.44 U/g, or 6.10 U/g instead of 3.66 U/g in Example 8, the fluid gel prepared by adding transglutaminase "Activa (registered trademark) TG" to 6.10 U/g instead of 3.66 U/g and then not stirring with a homomixer in Example 8, and the protein solution obtained according to the method described in Example 8 were measured using a laser diffraction-scattering particle size distribution measuring device LA-960 (HORIBA, LTD.).

The results thereof (D50 particle size) are shown in Table 7.

[Table 7]

| amount of TG added (U/g) | D50 (um) |
|---|---|
| 0.00 (protein solution) | 0.064 |
| 6.10 (no stirring) | 678 |
| 6.10 | 502 |
| 3.66 | 468 |
| 2.44 | 40 |
| 1.22 | 12 |

[Industrial Applicability]

The present invention provides a fluid gel which can impart good properties such as stickiness-free viscosity and a rich taste, good melt in mouth, foaming property, and the like to semi-solid foods, and which itself is superior in emulsifying property, and a production method thereof, emulsifiers and semi-solid foods containing the fluid gel, and the like. In addition, according to the production method, a fluid gel can be obtained without heating at a high temperature. Furthermore, by using the fluid gel, for example, low-calorie mayonnaise or whipped cream that has property closer to full-fat mayonnaise or whipped cream that uses high-fat milk fat can be obtained, even when low-fat raw materials are used. Therefore, the present invention is useful in the food industry.

This application is based on a patent application No. 2022-032720 filed in Japan (filing date: March 3, 2022), the contents of which are incorporated in full herein.

**Claims**

1.  A method for producing a fluid gel, comprising stirring a solution comprising a protein and transglutaminase.

2.  The method for producing a fluid gel according to claim 1, wherein a temperature during the stirring is a temperature at which the protein does not denature.

3.  The method for producing the fluid gel according to claim 1, wherein a stirring speed during the stirring is 100 rpm to 30,000 rpm.

4.  The method for producing a fluid gel according to claim 1, wherein the transglutaminase is used in an amount of 0.001 U to 1,000 U per 1 g of the protein.

5.  The method for producing a fluid gel according to claim 1, wherein the transglutaminase is added to the solution comprising the protein while stirring.

6.  The method for producing a fluid gel according to any one of claims 1 to 5, wherein the protein is a plant protein.

7.  The method for producing a fluid gel according to any one of claims 1 to 5, wherein the protein is an animal protein.

8.  A fluid gel comprising a protein treated with transglutaminase, the fluid gel having a median diameter of 0.1 $\mu$m to 1,000 $\mu$m.

9.  A fluid gel comprising a protein treated with transglutaminase and satisfying one or more of the following properties at 25°C:

    (1) coordination number in an angular frequency range of 0.1 to 100 rad/sec: 0.1 to 20
    (2) gel strength in an angular frequency range of 0.1 to 100 rad/sec: 0.1 Pas$^{1/z}$ to 100 Pas$^{1/z}$
    (3) viscosity coefficient in a shear rate range of 1 to 100/sec: 0.1 Pas$^n$ to 100 Pas$^n$.

10. The fluid gel according to claim 9, having a median diameter of 0.1 um to 1000 $\mu$m.

11. An emulsifier comprising the fluid gel according to any one of claims 8 to 10.

12. A semi-solid food comprising the fluid gel according to any one of claims 8 to 10.

13. The semi-solid food according to claim 12, which is yoghurt, mayonnaise or whipped cream.

14. A method for producing a semi-solid food, comprising mixing the fluid gel according to any one of claims 8 to 10 with a raw material for a semi-solid food.

15. The production method according to claim 14, wherein the semi-solid food is yoghurt, mayonnaise or whipped cream.

16. A method for improving the property of a semi-solid food, comprising mixing the fluid gel according to any one of claims 8 to 10 with a raw material for a semi-solid food.

[Fig. 1]

[Fig. 2]

[Fig. 3]

[Fig. 4]

[Fig. 5]

[Fig. 6]

[Fig. 7]

[Fig. 8]

[Fig. 9]

[Fig. 10]

[Fig. 11]

[Fig. 12]

[Fig. 13]

No. 1 No. 2 No. 3 No. 4 No. 5 No. 6

[Fig. 14]

[Fig. 15]

# EP 4 487 697 A1

<table>
<tr><td colspan="2" align="center"><b>INTERNATIONAL SEARCH REPORT</b></td><td>International application No.<br><br><b>PCT/JP2023/008032</b></td></tr>
</table>

**A. CLASSIFICATION OF SUBJECT MATTER**

*A23L 29/281*(2016.01)i; *A23C 9/137*(2006.01)i; *A23C 13/16*(2006.01)i; *A23J 3/00*(2006.01)i; *A23J 3/16*(2006.01)i; *A23L 5/00*(2016.01)i; *A23L 9/20*(2016.01)i; *A23L 27/60*(2016.01)i; *C12N 9/10*(2006.01)i; *C12P 21/04*(2006.01)i
FI: A23L29/281; A23C9/137; A23C13/16; A23J3/00; A23J3/16 501; A23L5/00 M; A23L9/20; A23L27/60 A; C12N9/10; C12P21/04

According to International Patent Classification (IPC) or to both national classification and IPC

**B. FIELDS SEARCHED**

Minimum documentation searched (classification system followed by classification symbols)

A23L29/281; A23C9/137; A23C13/16; A23J3/00; A23J3/16; A23L5/00; A23L9/20; A23L27/60; C12N9/10; C12P21/04

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS/FSTA/AGRICOLA (STN)

**C. DOCUMENTS CONSIDERED TO BE RELEVANT**

| Category* | Citation of document, with indication, where appropriate, of the relevant passages | Relevant to claim No. |
|---|---|---|
| X | GUO, Jian et al. Preparation of soy protein-based microgel particles using a hydrogel homogenizing strategy and their interfacial properties. Food Hydrocolloids. 2016, vol. 58, pages 324-334<br>    particularly, abstract, 2.2 Preparation of the microgel particles, 3.3. Microgel particles-stabilized oil-in-water emulsion | 1-4, 6, 8-11 |
| A | particularly, abstract, 2.2 Preparation of the microgel particles, 3.3. Microgel particles-stabilized oil-in-water emulsion | 5, 7, 12-16 |
| X | WEN, Xiaoyu et al. Transglutaminase induced gels using bitter apricot kernel protein: Chemical, textural and release properties. Food Bioscience. 2018, vol. 26, pages 15-22<br>    particularly, 2.3. Preparation of MTGase induced APC gels | 1-6, 8 |
| A | particularly, 2.3. Preparation of MTGase induced APC gels | 7, 9-16 |

☑ Further documents are listed in the continuation of Box C.   ☑ See patent family annex.

\* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **29 March 2023** | **18 April 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**

| International application No. |
| --- |
| **PCT/JP2023/008032** |

### C. DOCUMENTS CONSIDERED TO BE RELEVANT

| Category* | Citation of document, with indication, where appropriate, of the relevant passages | Relevant to claim No. |
| --- | --- | --- |
| X | BAYRAK, Meltem et al. Investigating casein gel structure during gastric digestion using ultra-small and small-angle neutron scattering. Journal of Colloid and Interface Science. 2021, vol. 594, pages 561-574 particularly, 2.2.1. Preparation of solubilized micellar casein solution and gels, fig. 3 | 1-5, 7-10 |
| A | particularly, 2.2.1. Preparation of solubilized micellar casein solution and gels, fig. 3 | 6, 11-16 |
| X | JP 2019-510514 A (OATLY AKTIEBOLAG) 18 April 2019 (2019-04-18) claims 1-34, examples | 1-6, 8-10, 12-16 |
| A | claims 1-34, examples | 7, 11 |
| X | JP 8-131119 A (AJINOMOTO COMPANY, INCORPORATED) 28 May 1996 (1996-05-28) claim 1, examples | 1-4, 7-16 |
| X | US 2021/0360940 A1 (INSTITUTE OF FOOD SCIENCE AND TECHNOLOGY, CHINESE ACADEMY OF AGRICULTURAL SCIENCES) 25 November 2021 (2021-11-25) claims 1-20, examples | 8-12, 14, 16 |
| A | claims 1-20, examples | 1-7, 13, 15 |
| X | CN 112890165 A (INSTITUTE OF FOOD SCIENCE AND TECHNOLOGY CAAS) 04 June 2021 (2021-06-04) claims 1-10, examples | 1-6, 8-11 |
| A | claims 1-10, examples | 7, 12-16 |

Form PCT/ISA/210 (second sheet) (January 2015)

**INTERNATIONAL SEARCH REPORT**
Information on patent family members

International application No.

**PCT/JP2023/008032**

| Patent document cited in search report | | | Publication date (day/month/year) | Patent family member(s) | | | Publication date (day/month/year) |
|---|---|---|---|---|---|---|---|
| JP | 2019-510514 | A | 18 April 2019 | US 2019/0110501 A1 claims 1-19, examples WO 2017/171601 A1 EP 3435781 A1 CN 109068684 A KR 10-2019-0003540 A | | | |
| JP | 8-131119 | A | 28 May 1996 | (Family: none) | | | |
| US | 2021/0360940 | A1 | 25 November 2021 | (Family: none) | | | |
| CN | 112890165 | A | 04 June 2021 | (Family: none) | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014512193 A
- JP 2003506061 A
- JP H03280834 A
- JP S6427471 A
- JP H06113844 A
- JP 2005144600 A

**Non-patent literature cited in the description**

- *Journal of the Japan Oil Chemists' Society*, 1970, vol. 19 (8), 826
- *Journal of the Japan Oil Chemists' Society*, 1979, vol. 28 (10), 781
- Chemical Dictionary. Morikita Publishing, 2009
- *RHEOLOGY TORONKAI KOEN YOSHISHU (Proceedings of the Rheology Symposium)*, 17 October 2018, vol. 66th, 322-323
- *J Biorheol*, 2018, vol. 32 (1), 9-14
- *Rheologica Acta*, 2001, vol. 40, 120-127